# EUROPEAN PATENT SPECIFICATION

(11) **EP 4 010 063 B1**
(45) Date of publication and mention of the grant of the patent: **15.11.2023**
(21) Application number: 20768233.7
(22) Date of filing: 21.09.2020
(51) Int. Cl.: A61N 1/04, A61N 1/36

(54) **NEUROSLEEVE FOR TUNING TREMOR SUPRESSION USING ELECTRICAL STIMULATION BY VAGUS NERVE STIMULATION OR DIRECT BRAIN STIMULATION**
NEURO-MANSCHETTE ZUR ANPASSUNG DER TREMORUNTERDRÜCKUNG DURCH ELEKTRISCHE STIMULATION DES VAGUSNERVS ODER DIREKTE HIRNSTIMULATION
NEURO-MANCHON POUR AJUSTER LA SUPPRESSION DES TREMBLEMENTS PAR STIMULATION ÉLECTRIQUE DU NERF VAGUE OU STIMULATION CÉRÉBRALE DIRECTE

(30) Priority: 26.06.2020 WO PCT/US2020/039802
(43) Date of publication of application: 15.06.2022
(73) Proprietor: Battelle Memorial Institute, Columbus, OH 43201 (US)
(72) Inventor: SHARMA, Gaurav, Lewis Center, OH 43035 (US); GANZER, Patrick, Columbus, OH 43201 (US)
(74) Representative: Kador & Partner Part mbB
(86) International application number: PCT/US2020/048748
(87) International publication number: WO 2021/262206

(56) References cited:
- EP-A1- 2 801 389
- WO-A1-2014/113813
- WO-A1-2019/126080
- WO-A2-2014/089266
- WO-A2-2018/098046
- US-A1- 2008 288 020
- US-A1- 2015 306 373
- US-A1- 2018 081 439
- US-A1- 2019 001 117
- US-A1- 2019 142 339

## Description

### BACKGROUND

The following relates to the neuromuscular tremor control arts, RF transmitter arts, RF receiver arts, RF transceiver arts, broadband RF transmitter, receiver, and/or transceiver arts, RF communications arts, and related arts.

Current treatments for pathological tremors (involuntary muscle tremors due to an underlying disease such as Parkinson's disease, essential tremor disorder, or so forth) are not effective in about one-quarter of the population. Tremor movements that can have a large disabling impact include elbow flexion/extension, forearm pronation/supination, and wrist flexion/extension. Current biomechanical loading-based methods for tremor suppression are not effective as this type of therapy often leads to distal-to-proximal migration of tremors. Current FES-based tremor suppression technologies cannot alleviate pronation/supination tremors due to difficulty in selectively targeting muscles. US Patent Application Publication 2019/001117 discloses a neuromodulation system for treatment of physiological disorders, having a control unit that controls nerve stimulation by stimulator, so that it is synchronized with predetermined physiological state detected by detector.

Certain improvements are disclosed herein.

### BRIEF SUMMARY

The invention is defined in claim 1. Further aspects and preferred embodiments are defined in the appended claims. Aspects, embodiments and examples of the present disclosure which do not fall under the scope of the appended claims do not form part of the invention and are merely provided for illustrative purposes. Furthermore, the methods presented in the present description are provided for illustrative purposes only and do not form part of the present invention.

### BRIEF DESCRIPTION OF THE DRAWINGS

Any quantitative dimensions shown in the drawing are to be understood as non-limiting illustrative examples. Unless otherwise indicated, the drawings are not to scale; if any aspect of the drawings is indicated as being to scale, the illustrated scale is to be understood as non-limiting illustrative example.
FIGURE 1 diagrammatically illustrates a tremor suppression device.
FIGURE 2 diagrammatically illustrates a tremor suppression process suitably performed by the tremor suppression processor of the device of FIGURE 1.
FIGURE 3 diagrammatically illustrates a tremor suppression process performed by Vagus Nerve Stimulation (VNS) or Direct Brain Stimulation (DBS) with the tremor suppression monitored using the garment of FIGURE 1.

### DETAILED DESCRIPTION

Some illustrative examples disclosed herein comprise a wearable myoelectric-enabled neuromuscular electrical stimulation (NMES) sleeve providing closed-loop control to discriminate between voluntary motion and tremor-induced motion and to suppress tremors with selective NMES stimulation of muscles, thereby providing suppression or attenuation of mild, moderate and severe tremors. In some variant examples, such an NMES sleeve is used in combination with vagus nerve stimulation (VNS) in conjunction with motion to suppress tremors or spasticity.

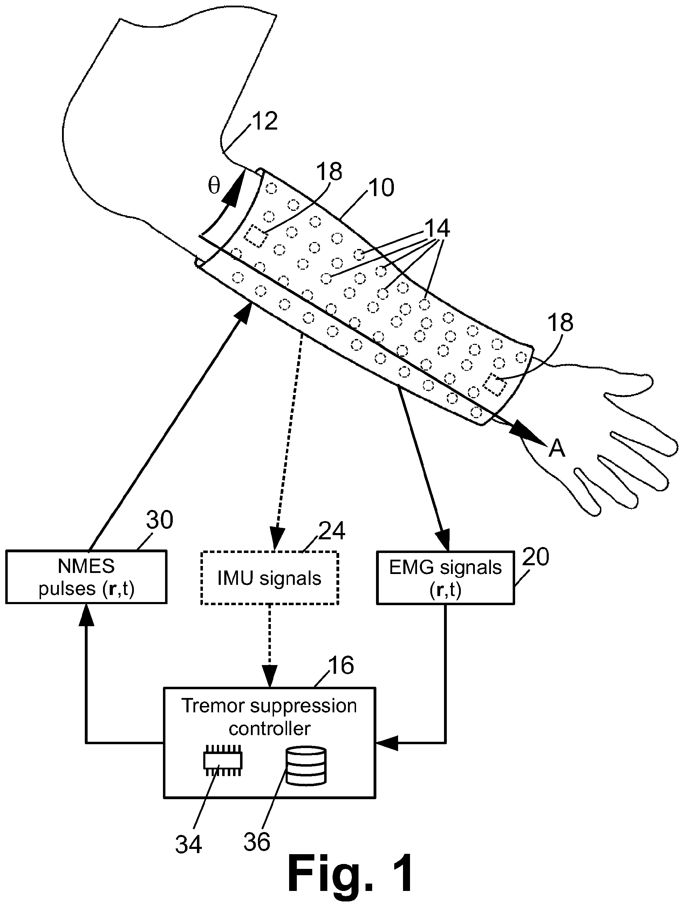

With reference to FIGURE 1, a garment **10** is wearable on an anatomical region **12,** and includes electrodes **14** contacting the anatomical region **12** when the garment is worn on the anatomical region. The illustrative garment is a sleeve **10** worn on an arm **12.** More generally, the garment may be made of a cloth, textile, leather, polyester, or other material, and is sized and shaped to be worn on the anatomical region for which tremor suppression therapy is to be provided. For example, the garment may be a sleeve that is sized and shaped to be worn on an arm, a leg, a wrist, an ankle, an arm and a wrist, or a leg and an ankle. The sizing is suitably patient-specific to account for different anatomies of different patients, or the garment may be designed to be adjustable for differences between patients - for example, the sleeve could employ a wrap-around arrangement with Velcro to be adjustably wrapped around arms of different diameters. In other examples, the anatomical region may be an arm, a leg, a wrist, an ankle, a hand, a foot, an arm and a wrist, a leg and an ankle, an arm and a wrist and a hand, a leg and an ankle and a foot, a wrist and a hand, or an ankle and a foot. Suitable garments for a hand would include, for example, a glove or mitten. Suitable garments for a foot would include, for example, a sock or boot. The glove, mitten, sock, or boot can be extended over the wrist or ankle to provide a garment for a wrist and hand or for an ankle and foot, or further extended to provide a garment for an arm and wrist and hand or for a leg and ankle and foot. These are merely non-limiting illustrative examples. The electrodes **14** are disposed on the inside of the garment **10** so as to contact the skin of the anatomical region **12** (FIGURE 1 illustrates the garment **10** as transparent so as to reveal the underlying electrodes **14,** but more typically the garment will be translucent or opaque), and are connected by wires (possibly woven into the garment), circuitry of flexible printed circuit boards, and/or so forth to an electrical connector (not shown) that connects with an electronic tremor suppression controller **16.** Alternatively, the electronic tremor suppression controller **16** can be integrated with the garment **10,** for example as a compact electronics package that is sewn onto or otherwise attached to the garment **10,** and the wires, printed circuitry, or the like connects the electrodes **14** directly with the attached electronic tremor suppression controller **16.** The electrodes **14** are designed to provide good electrical contact with the skin of the anatomical region **12.** For example, the electrodes **14** may be electrogel discs. Optionally, the garment may further include at least one Inertial Motion Unit (IMU) **18** (illustrative FIGURE 1 shows two IMUs **18,** one near each end of the illustrative sleeve garment **10**). The IMUs **18** may, for example, be accelerometers, gyroscopes, or the like.

By way of further non-limiting illustration, some suitable examples of the garment **10** wearable on the anatomical region **12** and including electrodes **14** contacting the anatomical region when the garment is worn on the anatomical region are described in Bouton et al., U.S. Pat. No. 9,884,178 issued February 6, 2018 and Bouton et al., U.S. Pat. No. 9,884,179 issued February 6, 2018.

The term "anatomical location" is used herein in describing operation of the tremor suppression device. The anatomical location is defined with respect to the anatomical region **12,** and each electrode **14** of the array of electrodes **14** of the garment **10** worn on the anatomical region **12** has a corresponding anatomical location. The anatomical region can be identified using a suitable coordinate system. The illustrative example of FIGURE 1 employs an anatomical coordinate system with an axial coordinate A and an azimuthal coordinate θ. The anatomical location (denoted herein as r) of any particular electrode **14** on the anatomical region **12** is given by its axial and azimuthal coordinates, that is, **r**=(A,θ). This provides a way to specify the anatomical location with high spatial resolution. Some examples may not require such high spatial resolution - in such cases, the anatomical location may be given by a designation such as "upper wrist location", "lower wrist location", or so forth, or by designation of a particular underlying muscle, such as a designated flexor muscle. The association of a particular electrode **14** with a particular anatomical location may be inherent in the design of the garment **10** (for example, if the garment is a glove then there is substantially no flexibility as regarding how the glove fits onto the hand (which is the anatomical region in this case) so that each electrode is inherently in a known anatomical location when the glove is placed onto the hand. In other examples, there may be enough variability as to how the garment **10** fits onto the anatomical region **12** so that some alignment is appropriate. For example, illustrative sleeve **10** might be worn in various angular positions in the azimuthal (θ) direction. In such cases, suitable garment positional alignment may be determined by the person fitting the garment onto the patient, and the positional alignment information is then input to the electronic tremor suppression controller **16.** In another approach, the garment may have some positioning index information, for example the illustrative sleeve **10** could include an alignment mark near the wrist end that is to be aligned with the medial vein of the forearm.

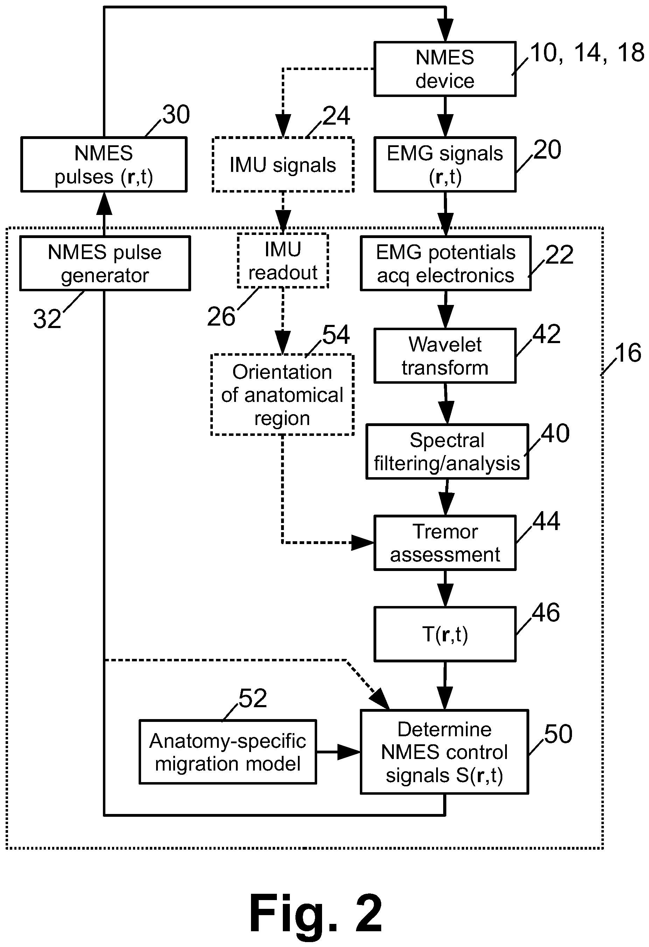

With continuing reference to FIGURE 1 and with further reference to FIGURE 2, the electronic tremor suppression controller **16** is configured to detect electromyography (EMG) signals **20** as a function of anatomical location (r) and time (t) using the electrodes **14.** Typically, the electrodes **14** are surface electrodes (e.g. electrogel discs) and measure surface EMG signals **20;** however, use of needle electrodes or the like for measuring intramuscular EMG signals is also contemplated. The EMG signal measurements are potential difference measurements between pairs of electrodes **14** acquired using any suitable voltmeter circuit or other EMG potentials acquisition electronics **22.** The anatomical location (**r**) of an EMG signal is suitably designated by the employed electrodes, e.g. as a midpoint between the anatomical locations of the electrodes of the pair. The EMG signals **20** also vary as a function of time (t) in accord with various muscle contraction/relaxation activity. Hence, the EMG signals may be designated as EMG(r,t). The EMG potentials acquisition electronics **22** preferably further include analog-to-digital (A/D) circuitry to convert the EMG signals **20** to digital signal values.

Optionally, the electronic tremor suppression controller **16** is further configured to receive IMU signals **24** with suitable IMU readout circuitry **26.** In one contemplated example, the IMUs **18** are commercially available triple-axis accelerometers that output the IMU signals **24** as digital signals, and the IMU readout circuitry **26** is designed to read the digital accelerometer signals.

The electronic tremor suppression controller **16** is further configured to generate neuromuscular electrical stimulation (NMES) pulses **30** using an NMES pulse generator **32.** By way of some non-limiting illustrative embodiments, some suitable NMES pulse waveforms may include monophasic and biphasic pulses with a voltage between 80 to 300 Volts inclusive or higher. In one example, the NMES pulse waveform is a monophasic pulse with a peak current of 0-20 mA which is modulated to vary strength of muscle contraction, frequency of 50 Hz, and a pulse width duration of 500 ms. Again, these are merely non-limiting illustrative examples.

With particular reference to FIGURE 1, the electronic tremor suppression controller **16** further includes an electronic processor **34,** such as a microprocessor or microcontroller (or two or more microprocessors or microcontrollers, e.g. a quad-CPU) and a non-transitory storage medium **36** (which again may comprise two or more media, possibly of different types). In some advantageous embodiments, the non-transitory storage medium **36** comprises a flash memory, solid-state drive (SSD), or other non-volatile electronic memory, although other types of media such as magnetic (e.g. a hard disk drive), optical (e.g. an optical disk) or so forth are additionally or alternatively contemplated. The non-transitory storage medium **36** stores firmware or software comprising instructions that are readable and executable by the electronic processor **34** to perform disclosed tremor suppression control operations including identifying tremors as a function of anatomical location (**r**) and time (t) based on the EMG signals; and applying NMES at one or more anatomical locations as a function of time using the electrodes **14** to suppress the identified tremors. Advantageously, because the garment **10** is spatially extended (e.g. running from the wrist to the elbow in the illustrative example of FIGURE 1, or in other examples further covering the wrist and/or elbow) the tremor suppression device can deliver therapeutic (i.e., tremor suppressing) NMES at specific anatomical locations at specific times. In particular, the disclosed tremor suppression device can suppress tremors in the presence of tremor migration.

With particular reference to FIGURE 2, the detected EMG signals as a function of anatomical location and time are analyzed to identify tremors as a function of anatomical location and time. This identification should exclude voluntary motions. One way to do so is to leverage the observation that frequencies of EMG signals associated with voluntary motions are usually 1 Hz or lower; whereas, EMG signals associated with tremors are usually 2 Hz or higher in frequency. Hence, in some examples, spectral filtering of the EMG signals is used to remove frequency components corresponding to voluntary motion. For example, a high-pass or band-pass filter can be applied with a lower cutoff frequency of between 1 Hz and 2 Hz inclusive.

Optionally, tremors may be classified on the basis of a frequency analysis. Most voluntary movements generate EMG signals at low (≤ 1 Hz) frequency, while the EMG frequencies of tremors varies from rest tremor (3-6 Hz), postural tremor (4 - 12 Hz), kinetic/intention tremor (2 - 5 Hz or 7 Hz). These filtering operations for removal of EMG signals associated with voluntary movements and optional tremor classification are represented in FIGURE 2 by the spectral filtering/analysis operation **40.** In some examples, the EMG signals are optionally initially converted to a wavelets representation using a wavelets transform **42.**

The resulting processed and filtered EMG signals are (at least predominantly) associated with tremors, and are then processed by a tremor assessment operation **44** to identify (and optionally classify) tremors. For example, a tremor may be identified as a spatially localized region **r** which, over some time, exhibits EMG signals at the tremor frequency range above some minimum threshold amplitude. Such tremors **46** are denoted herein as tremors T(**r**,t). In examples employing a high spatial resolution anatomical coordinate system such as the illustrative (A,θ) anatomical coordinate system of FIGURE 1, T(**r**,t) can be represented at high spatial resolution by denoting the tremor anatomical position **r** in (A,θ) coordinates. In other examples, tremors T(**r**,t) are given with the anatomical position r more coarsely designated by a designation such as "upper wrist location", "lower wrist location", or so forth, or by designation of a particular underlying muscle, such as a designated flexor muscle.

In an operation **50,** NMES control signals are determined for driving the NMES pulse generator **32** to generate the NMES pulses at designated anatomical locations r at times (t). In one approach, antagonistic NMES pulses are generated at the same anatomical location as that of the tremor T(**r**,t). By "antagonistic" it is meant that the NMES pulses induce muscular motion that is opposite that of the tremor. More generally, the appropriate NMES for suppressing tremors may be determined by adaptive training of an artificial neural network (ANN), support vector machine (SVM), or other machine learning (ML) component to tune the NMES response for a specific patient. For example, the ML component can be adaptively trained to produce an NMES that rapidly suppresses the tremor-related EMG signal at the anatomical location of the identified tremor.

However, the approach of generating the NMES pulses at the same anatomical location as that of the tremor may fail to account for tremor migration, which can occur spontaneously or in response to suppression of the tremor. For example, suppression of tremor migration in a distal part of a limb may result in the tremor migration toward a proximal part of the limb.

To address tremor migration, in some examples the operation **50** predicts the likely direction (and optionally rate) of tremor migration. In one approach, this is done using an anatomy-specific tremor migration model **52.** For example, it is common for a tremor starting in the wrist to migrate proximally toward the elbow or shoulder. Hence, in one example, the anatomy-specific tremor migration model **52** indicates that NMES responsive to a tremor identified in the wrist should be applied to the wrist, the elbow, and shoulder, with progressively lower NMES energy. (This assumes the garment **10** extends over the entire arm including at least a portion of the shoulder, over the elbow and over at least a portion of the wrist. On the other hand, if the garment **10** only covers the wrist through the elbow then the anatomy-specific tremor migration model **52** indicates that the NMES should be applied to the wrist, and the elbow, with lower NMES energy at the elbow compared with the wrist). The anatomy-specific tremor migration model **52** may be implemented based on first principles (e.g., knowledge that the tremor migration is usually in the distal-to-proximal direction for the arm or leg) or by training an ANN, SVM, or other ML component using adaptive training to produce an NMES that rapidly suppresses the EMG signal over the anatomical region (thereby incorporating any tremor migration into the objective function optimized by the ML).

In another approach for addressing tremor migration, the migration can be measured in real time. This takes advantage of the extended length of the garment **10** and the measurement of EMG(**r**,t) as a function of time. Using the sleeve garment **10** of FIGURE 1 as an example, if the tremor initiates near the wrist but then migrates toward the elbow, this would be directly measurable as the EMG(r,t) signal would progressively move up the arm in the direction toward the elbow. The direction and rate of tremor migration is measured, and the NMES is then be applied just ahead of (e.g. just proximal of, in the specific example) the tremor migration front.

Since tremor migration can occur in response to tremor suppression, the applied NMES signal may also be an input to the operation **50,** creating a feedback loop where the known location/strength of the NMES is used to predict the likely direction (and possibly also the likely rate) of migration, so that the NMES can be proactively adjusted to suppress the tremor including its expected migration. For example, the applied NMES signal can be an input to the ANN, MVM, or other ML component that is adaptively trained to produce the NMES to rapidly suppress the EMG signal over the anatomical region.

In addition, if the optional IMUs **18** are included, then the output of the IMU readout **26** can be used to determine the orientation **54** of the anatomical region. This, in turn, can be used to more precisely tailor the NMES to suppress the tremors. For the IMU **18** near the wrist in the example of FIGURE 1 can be used to determine the pronation/supination of the hand. This can be a further input to the ANN, MVM, or other ML component that produces the NMES, so that the adaptive training takes into account the pronation/supination of the hand.

In some examples, the same electrodes **14** are used to detect the EMG signals and also to deliver the NMES. This can be done, for example, if the NMES is delivered as pulses with some dead times between the pulses, and the EMG is measured during the dead times (i.e., time domain multiplexing, TDM, of the EMG detection and NMES delivery is employed). In other examples, the electrodes **14** are divided into: a first set of electrodes used to detect the EMG signals; and a second set of electrodes used to deliver the NMES. The electrodes of the two sets are preferably interspersed over the surface area of the anatomical region. While in principle this would allow simultaneous EMG detection and NMES delivery, the NMES is likely to interfere with the EMG detection so that employing EMG detection/NMES delivery TDM is again likely to be beneficial.

In the following, some further aspects are described.

The wearable sleeve can be worn on the arm and includes a) a multitude of electrodes that can record EMG activity of the underlying muscles, b) a multitude of electrodes that can provide transcutaneous electrical stimulation of the muscles (where the EMG recording and stimulation can be done on the same electrodes or on separate electrodes), and optionally c) IMUs for tracking hand/arm position in real time in 3D space.

In another aspect, an NMES system is provided that: 1. can apply stimulation parameters to evoke inhibitory muscle activity or antagonist muscle activity; 2. dynamically adjust stimulation patterns to account for pronation/supination based on IMU data; 3. Further include a nerve stimulation interface that can stimulate the vagus or other nerve branch to affect muscular and / or spinal physiology. Nerve stimulation combined with muscle (i.e. NMES) can potentially further modulate tremor and / or spasticity. The NMES system further includes a control algorithm that can: 1. Take input from the EMG electrodes and IMU sensors on the sleeve; 2. Make decisions based on the inputs to discriminate voluntary movements from tremor induced motion. Note: Most voluntary movements are performed at low (< 1 Hz) freq while freq of tremors varies from rest tremor (3-6 Hz), postural tremor (4 - 12 Hz), kinetic/intention tremor (2 - 5 Hz or 7 Hz); 3. Decode the type and location of tremor-induced muscle activity; 4. Initiate NMES or nerve stimulation to minimize the effects of tremor by stimulating the inhibitory muscle activity or antagonist muscle activity; 5. Take into account the distal to proximal migration of tremors under attenuation to optimize tremor attenuation.

Advantageously, tremor migration can be detected by a combination of EMG and/or IMU data. Therefore, a strategy can be employed to overcome tremor migration by slowly ramping up stimulation amplitude and changing stimulation pattern or optimizing stimulation parameters such as freq, pulse width etc. A genetic algorithm-based approach may be used to minimize the error between the desired state and current state.

In yet another aspect, an oscillator (such as a Matsuoka oscillator) model may be used to generate rhythmic/oscillatory output to enable stimulation of rhythmic muscle activity to overcome cyclic tremor oscillations.

As discussed, the disclosed approaches are well-suited for suppressing pathological tremors (that is, involuntary muscle tremors due to an underlying disease such as Parkinson's disease, essential tremor disorder, or so forth). In another contemplated example, the disclosed tremor suppression can be applied for control of epilepsy seizures. For example, a child with epilepsy may experience seizures while sleeping. The garment **10** monitors and reports activity and provides stimulation to suppress or control the impact of a seizure. Additionally or alternatively, the garment **10** could apply higher power via the electrodes **14** to provide functional electrical stimulation (FES) which causes movement of an anatomical part. For example, FES could be applied to drive an arm to a position of least harm for the person experiencing a seizure. In similar fashion, FES could be applied to preserve muscle elasticity and/or prevent bed sores.

In a seizure monitoring example (which is combinable with the seizure suppression and/or interventional FES just described), EMG acquired by the electrodes **14** of the garment **10** is suitably used to detect seizures, or other types of episodic behaviors such as Post-Traumatic Stress disorder (PTSD) attacks, autistic outbursts, or the like.

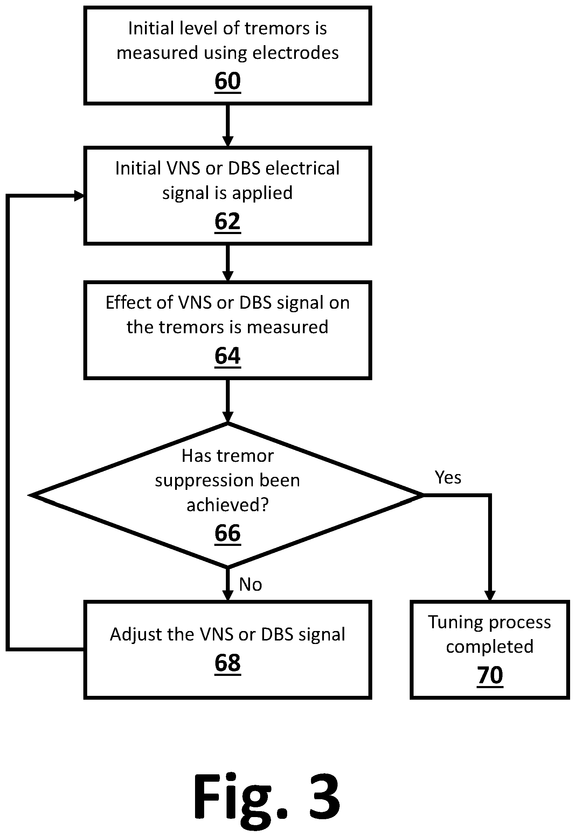

With reference to FIGURE 3, according to the present invention, EMG measurements performed using the garment **10** can be used to provide direct feedback on tremors during tuning of another type of tremor suppression therapy, such as Vagus Nerve Stimulation (VNS) or Direct Brain Stimulation (DBS). The garment **10** thus closes the loop on DBS or VNS to provide feedback on tremor suppression that is useful for optimizing the electrical signals applied via the VNS or DBS. For example, in the tuning approach of FIGURE 3, in an operation **60** the initial level of tremors is measured using the electrodes **14** of the garment **10.** In an operation **62,** a initial VNS or DBS electrical signal is applied, and in an operation **64** performed during and/or after the operation **62,** the effect of this VNS or DBS signal on the tremors is measured. At an operation **66** it is determined whether tremor suppression has been achieved. If not, then in an operation **68** the VNS or DBS electrical signal is adjusted, and the loop of operations **62, 64, 66, 68** is repeated until the VNS or DBS electrical signal is adjusted to adequately suppress tremors as measured by the operation **68,** at which point the tuning process is finished per block **70.**

In another contemplated embodiment, the garment **10** could be used with a coma patient, to keep the coma patient from having muscle atrophy, for example. Using FES to prevent muscle atrophy during severe paralysis (spinal cord injury, peripheral nerve transection, severe stroke, or so forth) would be done by stimulating a muscle that is completely unenervated to prevent atrophy, contractures, or other muscle degradation.

In another contemplated embodiment, the sleeve **10** can include a glove (not shown) to transfer sensation from extremities (e.g fingers) to higher up on the arm **12.** This can be beneficial for patients with diabetes or some neuropathy causing numbness or limited or lost feeling in the fingertips. The glove in this embodiment senses the location of objects on the hand and fingers, and electrodes on the sleeve **10** stimulate areas of the arm (where the patient still has feeling) with greater feeling. This might help with fine motor control. A similar approach can be used for the leg, using a legging as the garment **10** that includes a sock covering the foot, so as to transfer the sense of contact by the foot to a point further up on the leg.

In another contemplated embodiment, EMG decoding obtained by the electrodes **14** of the garment **10** can be used for sensory and proprioceptive rehabilitation. In this approach, visual sight of the hand (or other anatomical body part that is the target of the rehabilitation) is occluded, and the patient is asked to do various tasks. Over the course of the rehabilitation therapy, the amount of visual input that is received by the patient is controlled. For example: Ask the patient to perform different hand gestures, but only provide a virtual hand displayed on a computer monitor as visual feedback. The amount of information provided is slowly turned down (e.g., turn off different joints, et cetera). This can be used as a proprioceptive rehabiliation strategy. Optionally, the electrodes **14** can be used to apply stimulation to provide proprioceptive feedback of a limb. For example, a closed-loop technique to sense and provide certain stimulation patterns can be used to help the patient understand how the hand is oriented in space (or if the hand is opened / closed, holding an object, et cetera).

In one contemplated variant, transfer learning can be used to more efficiently train a left-handed sleeve **10** for a person who already has trained a right-handed sleeve (or vice versa). In another variant, a piezoelectric sensor can be integrated into the garment **10** and used to measure angles of deflection. In combination with IMU sensors, this could provide valuable positional information and help in controlling stimulation target to correct an intended motion.

In another contemplated embodiment, an HTML-like language could be developed for controlling neurophysical stimulation and sensing performed using the garment **10.**

## Claims

1. An apparatus for tuning tremor suppression, the apparatus comprising:
a garment (10) configured to be worn on an anatomical region (12) of a patient and including an array of electrodes (14) disposed on an inside the garment and contacting the anatomical region when the garment is worn on the anatomical region; and
an electronic controller (16) configured to:
receive measurements from the array of electrodes (14) disposed on the inside of the garment (10) of baseline tremors in the anatomical region (12) prior to the application of an electrical stimulation to the patient by Vagus Nerve Stimulation (VNS) or Direct Brain Stimulation (DBS);
during or after the application of the electrical stimulation by VNS or DBS, receive measurements from the array of electrodes (14) disposed on the inside of the garment of modified tremors in the anatomical region as function of time; and
provide tremor suppression feedback to the VNS or DBS based on the measurements of the baseline tremors and the measurements of the modified tremors as a function of time.

2. The apparatus of claim 1, wherein the measurements of the modified tremors are received during the application of the electrical stimulation by VNS or DBS..

3. The apparatus of any one of claims 1-2, wherein the garment (10) is a sleeve and the anatomical region (12) is an arm, a leg, a wrist, an ankle, an arm and a wrist, or a leg and an ankle.

4. The apparatus of any one of claims 1-2, wherein the anatomical region (12) is an arm, a leg, a wrist, an ankle, a hand, a foot, an arm and a wrist, a leg and an ankle, an arm and a wrist and a hand, a leg and an ankle and a foot, a wrist and a hand, or an ankle and a foot.

5. The apparatus of any one of claims 1-2, wherein the electrical stimulation to the patient is by VNS.

6. The apparatus of any one of claims 1-2, wherein the electronic controller (16) is configured to perform closed-loop feedback control of the application of the electrical stimulation to the patient by VNS or DBS by repeating the receiving of the modified tremors in the anatomical region as a function of time and the providing of the tremor suppression feedback to the VNS or DBS until tremor suppression is achieved.

## Patentansprüche

1. Vorrichtung zur Anpassung einer Tremorunterdrückung, wobei die Vorrichtung umfasst:
ein Kleidungsstück (10), das konfiguriert ist, um an einem anatomischen Bereich (12) eines Patienten getragen zu werden, und eine Anordnung von Elektroden (14) umfasst, die an einer Innenseite des Kleidungsstücks angeordnet sind und den anatomischen Bereich kontaktieren, während das Kleidungsstück an dem anatomischen Bereich getragen wird, und
eine elektronische Steuereinrichtung (16), die konfiguriert ist zum:
Empfangen von Messungen von der an der Innenseite des Kleidungsstücks (10) angeordneten Anordnung von Elektroden (14) in Bezug auf Baseline-Tremores in den anatomischen Bereich (12) vor der Anlegung einer elektrischen Stimulation an dem Patienten durch eine VNS (Vagus Nerve Stimulation) oder eine DBS (Direct Brain Stimulation),
während oder nach der Anlegung der elektrischen Stimulation durch eine VNS oder
DBS, Empfangen von Messungen von der an der Innenseite des Kleidungsstücks angeordneten Anordnung von Elektroden (14) in Bezug auf modifizierte Tremores in dem anatomischen Bereich als eine Funktion der Zeit, und
Vorsehen einer Tremorunterdrückung-Rückkopplung zu der VNS oder DBS basierend auf den Messungen der Baseline-Tremores und den Messungen der modifizierten Tremores als einer Funktion der Zeit.

2. Vorrichtung nach Anspruch 1, wobei die Messungen der modifizierten Tremores während der Anlegung der elektrischen Stimulation durch eine VNS oder DBS empfangen werden.

3. Vorrichtung nach Anspruch 1 oder 2, wobei das Kleidungsstück (10) eine Hülse ist und der anatomische Bereich (12) ein Arm, ein Bein, ein Handgelenk, ein Fußgelenk, ein Arm und ein Handgelenk oder ein Bein und ein Fußgelenk ist.

4. Vorrichtung nach Anspruch 1 oder 2, wobei der anatomische Bereich (12) ein Arm, ein Bein, ein Handgelenk, ein Fußgelenk, eine Hand, ein Fuß, ein Arm und ein Handgelenk, ein Bein und ein Fußgelenk, ein Arm und ein Handgelenk und eine Hand, ein Bein und ein Fußgelenk und ein Fuß, ein Handgelenk und eine Hand oder ein Fußgelenk und ein Fuß ist.

5. Vorrichtung nach Anspruch 1 oder 2, wobei die elektrische Stimulation für den Patienten mittels einer VNS bewerkstelligt wird.

6. Vorrichtung nach Anspruch 1 oder 2, wobei die elektronische Steuereinrichtung (16) konfiguriert ist zum Durchführen einer Rückkopplungssteuerung im geschlossenen Regelkreis der Anlegung der elektrischen Stimulation an dem Patienten durch eine VNS oder DBS durch das Wiederholen des Empfangens der modifizierten Tremores in dem anatomischen Bereich als einer Funktion der Zeit und des Vorsehens der Tremorunterdrückung-Rückkopplung zu der VNS oder DBS, bis eine Tremorunterdrückung erzielt wird.

## Revendications

1. Appareil pour régler la suppression de tremblements, l'appareil comprenant :
un vêtement (10) configuré pour être porté sur une région anatomique (12) d'un patient et comprenant un réseau d'électrodes (14) disposées sur un intérieur du vêtement et entrant en contact avec la région anatomique lorsque le vêtement est porté sur la région anatomique ; et
un régulateur électronique (16) configuré pour :
recevoir des mesures, provenant du réseau d'électrodes (14) disposées à l'intérieur du vêtement (10), de tremblements essentiels dans la région anatomique (12) avant l'application d'une stimulation électrique au patient par stimulation du nerf vague (SNV) ou stimulation cérébrale directe (SCD) ;
pendant ou après l'application de la stimulation électrique par SNV ou SCD, recevoir des mesures, provenant du réseau d'électrodes (14) disposées à l'intérieur du vêtement, de tremblements modifiés dans la région anatomique en fonction du temps ; et
fournir une rétroaction de suppression des tremblements à la SNV ou SCD sur la base des mesures des tremblements essentiels et des mesures des tremblements modifiés en fonction du temps.

2. Appareil selon la revendication 1, dans lequel les mesures des tremblements modifiés sont reçues pendant l'application de la stimulation électrique par SNV ou SCD.

3. Appareil selon l'une quelconque des revendications 1 et 2, dans lequel le vêtement (10) est un manchon et la région anatomique (12) est un bras, une jambe, un poignet, une cheville, un bras et un poignet, ou une jambe et une cheville.

4. Appareil selon l'une quelconque des revendications 1 et 2, dans lequel la région anatomique (12) est un bras, une jambe, un poignet, une cheville, une main, un pied, un bras et un poignet, une jambe et une cheville, un bras et un poignet et une main, une jambe et une cheville et un pied, un poignet et une main ou une cheville et un pied.

5. Appareil selon l'une quelconque des revendications 1 et 2, dans lequel la stimulation électrique au patient se fait par SNV.

6. Appareil selon l'une quelconque des revendications 1 et 2, dans lequel le régulateur électronique (16) est configuré pour effectuer une commande de rétroaction en boucle fermée de l'application de la stimulation électrique au patient par SNV ou SCD en répétant la réception des tremblements modifiés dans la région anatomique en fonction du temps et la fourniture de la rétroaction de suppression des tremblements à la SNV ou SCD jusqu'à ce que la suppression des tremblements soit accomplie.
